# EUROPEAN PATENT SPECIFICATION

(11) **EP 4 103 770 B1**
(45) Date of publication and mention of the grant of the patent: **21.01.2026**
(21) Application number: 21710346.4
(22) Date of filing: 12.02.2021
(51) Int. Cl.: A61L 15/44, B32B 5/24, B32B 15/14, A61L 15/00, A61Q 19/00, D01D 5/00, B32B 27/36, B32B 15/20, A61K 8/02, B32B 5/26, B32B 5/02, A61K 8/73, B32B 27/12, D01F 1/10, A61K 8/65

(54) **PATCH PRODUCT BASED ON NATURAL POLYMERS**
PATCH-PRODUKT AUF DER GRUNDLAGE VON NATÜRLICHEN POLYMEREN
PRODUIT PATCH À BASE DE POLYMÈRES NATURELS

(30) Priority: 13.02.2020 IT 202000002836; 05.01.2021 IT 202100000113
(43) Date of publication of application: 21.12.2022
(73) Proprietor: Bakel S.P.A., 33100 Udine (IT)
(72) Inventor: GREGORIS, Raffaella, 33100 Udine (IT); MANFREDINI, Stefano, 44049 Vigarano Mainarda (IT); VERTUANI, Silvia, 44123 Ferrara (IT)
(74) Representative: Petraz, Gilberto Luigi
(86) International application number: PCT/IB2021/051196
(87) International publication number: WO 2021/161256

(56) References cited:
- CN-A- 109 998 932
- GB-A- 2 484 319
- KR-Y1- 200 418 839
- US-A1- 2015 272 855
- US-A1- 2017 251 789

## Description

### FIELD OF THE INVENTION

The present invention concerns a product. More particularly, the invention concerns a patch product that can be absorbed by the skin and based on biocompatible polymer compounds, consisting of fibers, in particular nanofibers, preferably obtained by electrospinning, and packaged.

### BACKGROUND OF THE INVENTION

Products to be applied directly on the skin are widely known, which are formulated in various forms compatible with the skin and/or which are absorbed by the skin, such as for example, emulsions, creams, lotions, serums, gels, powders, oils, sun milks and suchlike, or other formulations.

These products, however, can sometimes be difficult to apply on the skin, sometimes requiring prolonged application times or a prolonged massage, in order to be completely absorbed into the dermis, and sometimes leaving residues on the fingers or hands or on any possible applicators used.

There are also products, in particular cosmetic products, applied by means of a physical support, generally fibrous, which facilitates their application on the skin, such as in particular make-up remover wipes, which are imbued with a special cleansing composition.

One disadvantage of this type of product is that, after use, the fibrous physical support, which does not have its own function except that of being a support, as well as the packaging, have to be disposed of.

Furthermore, known fibrous physical supports have a specific surface that does not allow suitable transport and release of the active ingredients.

The question also arises that, generally, cosmetic compositions for the skin, for example those to be spread, such as cream, or in general pastes, provide to use functional compounds for the skin and compounds that are exclusively functional for the structure of the formulation.

Functional compounds for the skin are partly or completely absorbed by the skin and bring benefits to the treated parts of the body. In general, this type of compound represents the so-called active ingredients.

The compounds that are exclusively functional for the structure of the formulation contribute to obtaining the composition to be applied, whether it be an emulsion, a serum, or fluid, an oleolyte, a water, or a gel.

The effects of a compound that is exclusively functional for the structure of the formulation may affect the storage method, or the consistency of the composition before its use and/or at the time of use, therefore when it is spread on the skin to be treated.

For example, a compound that is exclusively functional for the structure of the formulation can make the composition soft, fluid or viscous; it allows to obtain a gel and prevent the separation of the oily compounds from the aqueous ones in an emulsion. Furthermore, compounds exclusively functional for the structure of the formulation allow to preserve the composition, especially in the presence of water.

However, the problem arises that numerous compounds used, since they are functional for the structure of the formulation, do not bring any benefit to the skin, and indeed can even lead to a further deterioration of the treated skin. In fact, some of the compounds that are exclusively functional for the structure of the formulation remain indifferent to the skin, or they can cause, for example, further drying, pore closure, or development of allergies, dermatitis, or suchlike.

Among the compounds that are exclusively functional for the structure of the formulation, we can identify, for example, silicones, petrolatums (for example paraffin), alcohols, perfumes, stabilizers, preservatives, emulsifiers, the main function of which is to give the formulation of the composition a "silk effect" consistency in contact with the skin and/or to give stability to the oily parts, or to increase the viscosity of gel formulations and/or increase the emollient and humectant effect of emulsions.

For example, one problem that arises with these compounds exclusively functional for the structure of the formulation is the lack of skin compatibility, preventing the skin from breathing, also leading, for example, to its progressive drying, pore closure and/or greater sensitization.

It is also known that the presence of alcohols and preservatives on the one hand allow to preserve the composition, and on the other hand increase the sensitization of the skin in developing dermatitis, allergies, or similar pathologies.

US 2015/272855 describes a cosmetic product in the form of a sheet, consisting of nanofibers obtained by electrospinning a solution comprising a water-soluble polymer, in particular polyvinyl alcohol PVA and/or polyvinylpyrrolidone PVP, and a functional material in a solvent of water and/or alcohol. The sheet product is intended to be applied to the skin for a prolonged time, typically for hours.

US2017/251789 shows a sheet-shaped cosmetic product consisting of nanofibers of water-soluble polymer material obtained by electrospinning a corresponding solution to be electrospun. The electrospun polymer material is mainly polyvinyl alcohol PVA and/or polyvinylpyrrolidone PVP. The solution also comprises a functional compound, in particular a cross-linking agent, which can be an acid, an alcohol or a derivative of formaldehyde. This sheet product is also intended to be left to act on the skin for a prolonged time.

GB2484319 on the contrary describes electrospun fibers based on honey and a biocompatible polymer, which can for example comprise a polysaccharide or a synthetic polymer such as polyethylene oxide PEO, polyethylene terephthalate PET or polypropylene PP. The fibers described have an antimicrobial effect and can be used to treat skin wounds.

There is therefore a need to perfect a product that can overcome at least one of the disadvantages of the state of the art.

In particular, one purpose of the present invention is to provide a product that can be applied simply and quickly to the skin.

Another purpose of the present invention is to provide a product that does not have a physical support to be discarded after use.

Another purpose is to provide a product that has a specific surface such as to optimize the transport and release of active ingredients, if there are any.

The Applicant has devised, tested and embodied the present invention to overcome the shortcomings of the state of the art and to obtain these and other purposes and advantages.

### SUMMARY OF THE INVENTION

The present invention is set forth and characterized in the independent claims. The dependent claims describe other characteristics of the present invention or variants to the main inventive idea.

In accordance with the above purposes, a patch product has been produced which overcomes the limits of the state of the art and eliminates the defects present therein.

According to some embodiments, there is provided, in particular, a patch product, to be applied preferentially on the skin, made up of a membrane substrate suitable to be absorbed by the skin. The membrane substrate is electrospun. The electrospun substrate is made up of at least one fiber, preferably nanofiber, in particular in the form of non-woven fabric.

The membrane substrate contains one or more active ingredients, selected from cosmetic active ingredients, pharmaceutical active ingredients and nutritional active ingredients. With the expression "active ingredients" we mean, here and in this description, compounds that are functional for the body or parts of it, which are absorbed in part, or completely, by the skin and bring benefits to the parts of the body being treated.

The electrospun fiber also comprises pullulan and/or alginate, as such compound has been used as an electrospinning promoter during the eletrospinning process that led to the obtention of the electrospun fiber.

According to some embodiments, the substrate is based on a biocompatible polymer material, suitable to be electrospun, selected from a group consisting of polysaccharides, collagen, gelatin, albumin, elastin and their derivatives. Favorably, the polysaccharide is selected from a group consisting of xanthan gum, pectins, chitin, chitosan, dextran, carrageenan, guar gum, agar, cellulose derivatives, starch, gelatin, β-glucans, glycosaminoglycans, mucopolysaccharides, water-soluble polysaccharides and their derivatives.

Preferably, the cellulose derivatives are selected from hydroxypropyl methylcellulose HPMC, hydroxypropyl cellulose HPC, hydroxyethyl cellulose HEC, sodium carboxymethyl cellulose Na-CMC. Glycosaminoglycans GAGs or mucopolysaccharides can be selected from chondroitin sulfate, dermatan sulfate, heparin, heparan sulfate and hyaluronic acid HA. The water-soluble polysaccharides can be selected from galactomannans, xylans, gum arabic, gum ghatti, glucomannan, acemannan, soluble dietary fiber, glycogen, amylose and polysaccharides derived from plants, bacteria and fungi.

According to some embodiments, the active ingredient is integrated into the membrane substrate. Alternatively, it is possible to provide that the active ingredient is inserted into the three-dimensional structure of the membrane substrate.

According to some embodiments, the membrane substrate comprises linear and/or cross-linked hyaluronic acid. Advantageously, the membrane substrate is made both with linear hyaluronic acid and also with cross-linked hyaluronic acid.

Preferably, hyaluronic acid has a molecular weight of less than 10,000 Da. This molecular weight is advantageous for the complete absorbability of the hyaluronic acid in the skin.

One advantage of the patch product according to the embodiments described here is that it is completely absorbed by the skin, together with the active ingredient present, for example a cosmetic, pharmaceutical or nutritional active ingredient. In addition to being complete, the absorption is also fast, it has been verified that the total absorption of a patch product according to the invention occurs in just a few minutes from its application on the skin.

Another advantage is that the form in which the patch product is presented does not imply formulations that require the presence of compounds exclusively functional for the structure, since the active ingredients are carried and conveyed by the membrane substrate and the latter is completely biocompatible and absorbable by the skin, without causing any risk or damage to the skin itself.

Another advantage of the cosmetic, pharmaceutical or nutritional product lies in the possibility of reaching topical concentrations of the compound of interest much higher than those obtainable with traditional formulations. It is practically impossible to reach high concentrations, even with polysaccharides, collagen, gelatin, albumin, elastin and their low molecular weight derivatives, since the viscosity of the product increases too much and it is not possible to exceed 5-10% by weight. With the present composition, it is possible to obtain cosmetic products that allow to apply concentrations up to 50% by weight of the compound of interest on the skin.

According to another aspect, the present invention also concerns an article, comprising a patch product as indicated above disposed on a support base.

Preferably, the article comprises a wrapping, which in turn comprises the support base, inside which the patch product is present.

Advantageously, the wrapping also comprises a coating layer that acts as protection for the patch product present on the support base. The coating layer and the support base are reciprocally joined in order to form an internal space that houses the patch product. More preferably, the support base and the coating layer are reciprocally heat-sealed.

Advantageously, the support base and the coating layer are heat-sealable. Preferably, they are made of PBSA (polybutylene succinate adipate) or PLA (polylactic acid).

### DETAILED DESCRIPTION OF SOME EMBODIMENTS

We will now refer in detail to the possible embodiments of the invention, of which one or more examples are shown in the attached drawings, as a non-limiting example. The phraseology and terminology used here is also for the purposes of providing non-limiting examples.

Unless otherwise defined, all the technical and scientific terms used here and hereafter have the same meaning as commonly understood by a person with ordinary experience in the field of the art to which the present invention belongs. Even if methods and materials similar or equivalent to those described here can be used in practice and in the trials of the present invention, the methods and materials are described hereafter as an example. In the event of conflict, the present application shall prevail, including its definitions. The materials, methods and examples have a purely illustrative purpose and shall not be understood restrictively.

All measurements are carried out, unless otherwise indicated, at 25°C (room temperature) and at atmospheric pressure. All temperatures, unless otherwise indicated, are expressed in degrees Celsius.

All percentages and ratios indicated here are understood to refer to the weight of the total composition (w/w), unless otherwise indicated.

All percentage intervals reported here are supplied with the provision that the sum with respect to the overall composition is 100%, unless otherwise indicated.

All the intervals reported here shall be understood to include the extremes, including those that report an interval "between" two values, unless otherwise indicated.

The present description also includes the intervals that derive from overlapping or uniting two or more intervals described, unless otherwise indicated.

The present description also includes the intervals that can derive from the combination of two or more values taken at different points, unless otherwise indicated.

Where water is mentioned, we mean distilled water, unless otherwise specified.

Embodiments described here concern a patch product, preferably to be applied on skin, comprising an electrospun membrane substrate which is made up of at least one electrospun fiber, preferably nanofiber, in the form of non-woven fabric. This form is obtained by progressively depositing the fiber, during the electrospinning, on a support base. The fiber is progressively deposited on several layers which gradually overlap. It is possible to provide more fibers, which can for example be delivered simultaneously on the same support.

The electrospinning occurs by feeding a specific composition under an electric field, through an electrospinning head. The fiber at exit from the electrospinning head is then deposited on a support. The non-woven fabric form can be obtained by means of a relative movement between the electrospinning head and the support.

Preferably, the fiber is continuous, with a homogeneous composition and with a substantially smooth surface. The fiber is also preferably free of defects, that is, free from accumulations or drops of substrate that can form during the electrospinning. Advantageously, the electrospun fiber has a diameter of the order of the nanometer and micrometer, but in any case smaller than 100 µm, more preferably smaller than 50 µm, even more preferably smaller than 25 µm, at most of the order of 10 µm. The fibers of the substrate can have diameters starting from tens of nanometers, for example 50 nm. It should be noted that the diameter remains substantially unchanged along the entire fiber. By substantially unchanged diameter we mean that the diameter can undergo variations which however do not exceed 30% of the average value measured.

Fibers with these diameters have a greater surface/volume ratio than known fibers, which typically have a diameter greater than 100 µm, as well as greater mechanical and structural properties that offer greater efficiency and performance than industry standards. The large specific surface of the fibers obtained makes them particularly suitable to transport and release active ingredients. The nanofibers obtained allow to have a specific surface between 1 and 30 m²/g, preferably 2 and 20 m²/g.

The membrane substrate is made of a biocompatible polymer material suitable to be electrospun selected from xanthan gum, pectin, chitin, chitosan, dextran, carrageenan, guar gum, agar, hydroxypropyl methylcellulose HPMC, hydroxypropyl cellulose HPC, hydroxyethyl cellulose HEC, sodium carboxymethyl cellulose Na-CMC, albumin, starch, gelatin, collagen, elastin, β-glucans, chondroitin sulfate, dermatan sulfate, heparin, heparan sulfate, hyaluronic acid HA, galactomannans, xylans, gum arabic, gum ghatti, glucomannan, acemannan, soluble dietary fibers, glycogen, amylose and polysaccharides derived from plants, bacteria and fungi and their derivatives.

These compounds or classes of compounds have, as their property, the possibility of modifying the viscosity of liquids, which makes them suitable to form regular electrospun fibers with good mechanical and absorption properties. They are also all biocompatible, of natural origin, and can be used in the food, pharmaceutical and/or cosmetic sectors.

In particular, xanthan gum, dextran, carrageenan, Na-CMC, starch, gelatin and gum ghatti are used as a thickener, stabilizer and possibly also as a gelling agent in the food sector. In addition, xanthan gum is used as a stabilizer for suspensions and emulsions in the pharmaceutical and cosmetic sectors, guar gum is also used as a thickener and gelling agent in the pharmaceutical and cosmetic sectors, carrageenan is used as an inactive excipient in the pharmaceutical sector. Dextran is also used as a thickener in the pharmaceutical sector.

Chitosan is used in the food sector, in low-calorie diets, and in the pharmaceutical sector as an excipient, in particular for products to be inhaled. Pectin, on the other hand, is used as a gelling agent in the food sector and as a dietary and probiotic agent in the pharmaceutical sector.

Agar, galactomannans and glucomannan are used as a gelling agent in the nutritional sector.

Among cellulose derivatives, HPMC is used as a stabilizer and viscosity regulator in the food sector, and as collyrium or excipient for oral medicines in the pharmaceutical sector. HPC is used as a food additive, and as collyrium or binder for tablets in the pharmaceutical sector. HEC is used as a thickener and gelling agent in the pharmaceutical and cosmetic sectors.

β-glucans are used as dietary fiber, as are xylans. Chondroitin sulfate is used as a food supplement, and also in the treatment of osteoarthritis symptoms. Dermatan sulfate, heparin and heparan sulfate are known as anticoagulants in the pharmaceutical sector.

Gum arabic is used in the food sector as a stabilizing excipient and viscosity modifier. Acemannan, on the other hand, is known in the pharmaceutical sector for its immunostimulant properties.

It should be noted that some of these compounds have their own functions in the cosmetic, pharmaceutical or food sectors, such as for example starch, elastin, hyaluronic acid, heparin, collagen, pectin, β-glucans, chondroitin sulfate, dermatan sulfate, heparan sulfate and their derivatives, among others. It is therefore advantageous to electrospin these compounds, since the application of the corresponding fibers will allow to apply these compounds in higher doses than known solutions, to the advantage of their greater effectiveness.

The electrospun fiber also comprises pullulan and/or alginate, which are used as an electrospinning promoter during the production of the fiber. Advantageously, pullulan and/or alginate is present in a (electrospun compound):promoter proportion preferably comprised between 10:1 and 1:10, more preferably between 4:1 and 1:7, even more preferably between 3:1 and 1:6 by weight. Pullulan is the preferred spinning promoter, since it allows to obtain the best results in terms of the structure of the electrospun fiber.

According to some embodiments, the membrane substrate is made up of hyaluronic acid. Preferably, the substrate comprises linear and/or cross-linked hyaluronic acid. Advantageously, the substrate is made with both linear hyaluronic acid and also cross-linked hyaluronic acid, with linear HA:cross-linked HA proportions ranging from 1:99 to 99:1. Such a mixture allows to modulate the rigidity of the yarn obtained, as well as the three-dimensional structure of the non-woven fabric film.

The hyaluronic acid can have a high mass, for example of the order of a million Dalton or even more, or alternatively have a low mass, typically of the order of 10,000 Dalton or less. The latter form is preferred, since hyaluronic acid with a molecular mass lower than or equal to 10000 Da, in the electrospun form, once placed in contact with the skin is completely absorbable and is able to penetrate the stratum corneum, reaching the innermost layers where, thanks to its properties such as hygroscopicity, viscoelasticity and structural function, it is able to modulate the hydration of the tissues, the osmotic balance and the physical properties of the ECM.

Advantageously, the action of absorption and penetration into the skin of the membrane substrate also allows to convey toward the skin active ingredients associated with the membrane support.

In particular, according to some embodiments, the patch product also comprises at least one active ingredient, selected from cosmetic active ingredients, pharmaceutical active ingredients and nutritional active ingredients, which is associated with the membrane substrate.

It should be noted that the active ingredients can have various types of function, regardless of their field of action.

The cosmetic active ingredient can be of the following types: anti-seborrheic (e.g. sebacic acid, azelaic acid), anti-sebum (e.g. coal powder), antimicrobial (e.g. climbazole, piroctone olamine), antioxidant (e.g. ascorbic acid, tocopherol, coenzyme Q10, resveratrol, glutathione), antiperspirant (e.g. aluminum chlorohydrate, aluminum sesquichlorhydrate), astringent (e.g. Citrus aurantifolia flower extract, calcium lactate), whitener (e.g. glabridin, ammonium persulfate), make-up remover (e.g. sodium cocoyl glutamate), deodorant (e.g. triethyl citrate, zinc ricinoleate), exfoliant (e.g. glycolic acid, malic acid, mandelic acid), flavorings (e.g. citral, honey), fragrance (e.g. d, 1-limonene, coumarin), humectant (e.g. glycerin, propanediol), keratolytic (e.g. chloroacetic acid, salicylic acid), moisturizer (e.g. Aloe arborescens leaf extract), perfuming (e.g. geraniol, linalool), emollient (e.g. triolein, squalene), refreshing (e.g. menthol, menthyl lactate), skin moisturizer (e.g. panthenol, allantoin), skin protection (e.g. sphingolipids, zinc oxide), smoothing (e.g. Ricinus communis seed oil), soothing (e.g. extracts of Hamamelis virginiana, Chamomile recutica extracts, bisabolol) or tonic (e.g. arnica montana, Capsicum frutescens extract), UV filter (e.g. methylene bis-benzotriazolyl tetramethylbutylphenol, ethylhexyl methoxycinnamate, caffeine, theine, theobromine, theophylline).

The pharmaceutical active ingredient can be of the following types: 5-alpha-reductase inhibitor (e.g. finasteride), 5-aminosalicylates (e.g. mesalamine), 5HT3 receptor antagonist (e.g. ondansetron), ACE inhibitor with calcium channel blocker (e.g. amlodipine/benazepril), ACE inhibitor with thiazides (e.g. hydrochlorothiazide), adamantane antivirals (e.g. amantadine), adrenal corticosteroid inhibitor (e.g. aminoglutethimide), adrenergic bronchodilators (e.g. albuterol), hypertensive agent (e.g. hypertensive agent) pulmonary hypertension agent (e.g. treprostinil), aldosterone receptor antagonist (e.g. spironolactone), alkylating agent (e.g. cyclophosphamide), allergens (e.g. house dust mite allergen extracts), alpha-glucosidase inhibitor (e.g. miglitol), amoebicides (e.g. metronidazole), aminoglycosides (e.g. tobramiycin), aminopenicillins (e.g. amoxicillin), aminosalicylates (e.g. aminosalicylic acid), AMPA receptor antagonist (e.g. perampanel), amylin analogues (e.g. pramlintida), analgesics (e.g. acetaminophen), androgenic and anabolic steroids (e.g. testosterone), enzyme inhibitor converting angiotensin (e.g. ramipril), angiotensin II inhibitor with calcium channel blocker (e.g. amlodipine/olmesartan), angiotensin II inhibitor with thiazides (e.g. hydrochlorothiazide/olmesartan), angiotensin receptor blockers (e.g. valsartan), inhibitor of angiotensin and neprilysin receptor blockers (e.g. sacubitril/valsartan), anorectal preparations (e.g. hydrocortisone/pramoxin), anorexiants (e.g. phentermine), antacids (e.g. magnesium hydroxide), anthelmintics (e.g. pyrantel), anti-angiogenic ophthalmic agent (e.g. aflibercept), anti-CTLA-4 monoclonal antibody (e.g. ipilimumab), anti-PD-1 monoclonal antibody (e.g. nivolumab), antiadrenergic agent (central) with thiazides (e.g. hydrochlorothiazide/methyldopa), antiadrenergic agent (peripheral) with thiazides (e.g. polythiazide/prazosin), centrally acting antiadrenergic agent (e.g. guanfacine), peripherally acting antiadrenergic agent (e.g. tamsulosin), antiandrogens (e.g. enzalutamide), antianginal agent (e.g. nitroglycerin, e.g. diphylline/guaifenesin), antibiotics (e.g. metronidazole), antibiotics/antineoplastics (e.g. doxorubicin), anticholinergic antiemetics (e.g. diphenhydramine), anticholinergic antiparkinsonian agent (e.g. procyclidine), anticholinergic bronchodilators (e.g. tiotropium), anticholinergics/antispasmodics (e.g. hyoscyamine), anticoagulant agent (e.g. phytonadione), anticonvulsants (e.g. lacosamide), antidepressant (e.g. bupropion), antidiarrheal (e.g. loperamide), antidiuretic hormone (e.g. desmopressin), antidote (e.g. naltrexone dronabinol), antifungal (e.g. griseofulvin), antigonadotropic agent (e.g. g. danazol), antigout agent (e.g. colchicine), antihistamine (e.g. cetirizine), anti-hyperlipidemic agent and combinations (e.g. ezetimibe/simvastatin), antihyperuricemic agent (e.g. febuxostat), antimalarial (e.g. doxycycline), antimalarial combination, antimalarial quinoline (e.g. hydroxychloroquine), antimanic agent (e.g. lithium), antimetabolite (e.g. capecitabine), anti-migraine agent (e.g. rizatriptan), antineoplastic (e.g. isotretinoin), antineoplastic combination (e.g. letrozole/ribociclib), antineoplastic detoxifying agent (e.g. amifostine), antineoplastic interferon (e.g. interferon alfa-2b), antipseudomonal penicillin (e.g. carbenicillin), antipsoriatic agent (e.g. acitretin), antipsychotic agent (e.g. haloperidol), antirheumatic (e.g. adalimumab), antiseptic and germicidal agent, antithyroid agent (e.g. potassium iodide), antitoxin and antiviral (e.g. antivenin (crotalidae) polyvalent), antitussive (e.g. dextromethorphan), antiviral booster (e.g. ritonavir), antiviral interferon (e.g. peginterferon alfa-2a), aromatase inhibitor (e.g. anastrozole), atypical antipsychotic (e.g. aripiprazole), azole antifungal (e.g. fluconazole), bacterial vaccine (e.g. 13-valent pneumococcal vaccine), barbiturate anticonvulsant (e.g. primidone), barbiturate (e.g. phenobarbital), BCR-ABL tyrosine kinase inhibitor (e.g. imatin), benzodiazepine anticonvulsant (e.g. diazepam), benzodiazepine (e.g. clonazepam), beta blocker with thiazides (e.g. bisoprolol/hydrochlorothiazide), beta-lactamase inhibitor (e.g. clavulanic acid), bile acid sequestering agent (e.g. colesevelam), bisphosphonate (e.g. zoledronic acid), BTK inhibitor (e.g. ibrutinib), calcimimetic (e.g. cinacalcet), calcineurin inhibitor (e.g. tacrolimus), calcitonin, agent calcium channel blocker (e.g. verapamil), anticonvulsant carbamate (e.g. felbamate), carbapenema (e.g. doripenem), carbapenema/beta-lactamase inhibitor (e.g. meropenem/vaborbactam), anticonvulsant carbonic anhydrase inhibitor (e.g. topiramate), carbonic anhydrase inhibitor (e.g. acetazolamide), cardiac stressing agent (e.g. regadenoson), cardioselective beta-blockers (e.g. nebivolol), catecholamines (e.g. epinephrine), CD20 monoclonal antibody (e.g. ocrelizumab), CD30 monoclonal antibody (e.g. brentuximab), CD33 monoclonal antibody (e.g. gemtuzumab), CD38 monoclonal antibody (e.g. CD52 monoclonal), (e.g. alemtuzumab), CDK 4/6 inhibitor (e.g. palbociclib), cephalosporins/beta-lactamase inhibitor (e.g. avibactam/ceftazidime), cerumenolytics (e.g. carbamide peroxide), combination of CFTR (e.g. ivacaftor/lumacaftor), CFTR enhancer (e.g. ivacaftor), CGRP inhibitor (e.g. erenumab), chelating agent (e.g. deferasirox), chemokine receptor antagonist (e.g. maraviroc), chloride channel activator (e.g. lubiprostone), cholesterol absorption inhibitor (e.g. ezetimibe), cholinergic agonist (e.g. cevimeline), cholinergic muscle stimulants (e.g. pyridostigmine), cholinesterase inhibitor (e.g. donepezil), central nervous system stimulant (e.g. Phentermine), colony stimulating factor (e.g. Filgrastim), contraceptive (e.g. Levonorgestrel), corticotropin, coumarins and indandione (e.g. Warfarin), cox-2 inhibitor (e.g. Celecoxib), decongestant (e.g. pseudoephedrine), diarylquinoline (e.g.), dibenzazepine anticonvulsant (e.g. carbamazepine), digestive enzyme (e.g. lactase), dipeptidyl peptidase 4 inhibitor (e.g. sitagliptin), dopaminergic antiparkinsonian agent (e.g. ropinirole), drug used in alcohol dependence (e.g. acamprosate), echinocandin (e.g. caspofungin) inhibitor (e.g. erlotinib), estrogen receptor antagonist (e.g. fulvestrant), estrogen (e.g. estradiol), expectorant (e.g. guaifenesin), factor Xa inhibitor (e.g. rivaroxaban), fatty acid derivative anticonvulsant (e.g. divalproex sodium), fibric acid derivative (e.g. fenofibrate), first generation cephalosporins (e.g. cephalexin), fourth generation cephalosporins (e.g. cefepime), gallstone solubilizing agent (e.g. ursodiol), gamma-aminobutyric acid analogue (e.g. gabapentin), gamma-aminobutyric acid reuptake inhibitor (e.g. tiagabine), general anesthetic (e.g. propofol), GI stimulant (e.g. metoclopramide), glucocorticoids (e.g. budesonide), glucose elevating agent (e.g. glucagon), glycopeptide antibiotic (e.g. vancomycin), glycoprotein platelet inhibitor (e.g. tirofiban), glycylcycline (e.g. tigecycline), gonadotropin releasing hormone (e.g. leuprolide), gonadotropin releasing hormone antagonist (e.g. elagolix), gonadotropin (e.g. chorionic gonadotropin) group I antiarrhythmic (e.g. phenytoin), group II antiarrhythmic (e.g. propranolol), group III antiarrhythmic (e.g. dronedarone), group IV antiarrhythmic (e.g. verapamil), group V antiarrhythmic (e.g. digoxin), growth hormone receptor blocker (e.g. pegvisomant), growth hormone (e.g. somatropin), guanylate cyclase-C agonist (e.g. linaclotide), H. pylori eradication agent (e.g. bismuth subcitrate potassium/metronidazole/tetracyclines), H2 antagonist (e.g. ranitidine), hedgehog pathway inhibitor (e.g. vismodegib), heparin antagonist (e.g. protamine), HER2 inhibitor (e.g. neratinib), herbal product (e.g. 5-hydroxytryptophan, aloe vera), histone deacetylase inhibitor (e.g. romidepsin), hormone/antineoplastic (e.g. medroxyprogesterone), hydantoin anticonvulsant (e.g. phenytoin), hydrazide derivative (e.g. isoniazid), immunoglobulin, impotence agent (e.g. sildenafil), mimetic of incretin (e.g. liraglutide), inotropic agent (e.g. digoxin), insulin and derivatives (e.g. insulin glargine), insulin-like growth factor (e.g. mecasermin), interferon (e.g. interferon beta-1a), interleukin inhibitor (e.g. dupilumab), interleukin (e.g. aldesleukin), iron product (e.g. ferrous sulfate), ketolide (e.g. telithromycin), laxative (e.g. bisacodyl), leprostatic (e.g. clofazimine), leukotriene modifier (e.g. montelukast), lincomycin derivative (e.g. clindamycin), loop diuretic (e.g. furosemide), lysosomal enzyme (e.g. imiglucerase), macrolide (e.g. azithromycin), mast cell stabilizer (e.g. cromolyn), meglitinide (e.g. repaglinide), melanocortin receptor agonist (e.g. bremelanotide), methylxanthine (e.g. theocortic) mineral corticoid (e.g. fludrocortisone), mineral and electrolyte (e.g. citric acid/potassium citrate), various antivirals (e.g. baloxavir marboxil), various anxiolytics, sedatives and hypnotics (e.g. zolpidem), various bone resorption inhibitors (e.g. denosumab), various cardiovascular agents (e.g. midodrine), various agents of the central nervous system (e.g. dalfampridine), various coagulation modifiers (e.g. tranexamic acid), various diuretics (e.g. pamabrom), various agents of the genitourinary tract (e.g. phenazopyridine), various gastrointestinal agents (e.g. misoprostol), various metabolic agents (e.g. burosumab), various respiratory agents (e.g. alpha 1-proteinase inhibitor), various topical agents (e.g. sodium hyaluronate), various vaginal agents (e.g. estradiol), mitotic inhibitor (e.g. vincristine), monoamine oxidase inhibitor (e.g. phenelzine), mouth and throat product (e.g. fluoride), mTOR inhibitor (e.g. everolimus), mucolytic (e.g. acetylcysteine), multikinase inhibitor (e.g. sorafenib), combination of narcotic analgesics (e.g. buprenorphine/naloxone), narcotic analgesic (e.g. fentanyl), natural penicillin (e.g. penicillin v potassium), neuraminidase inhibitor (e.g. oseltamivir), neuronal potassium channel openers (e.g. ezogabine), new generation cephalosporins (e.g. ceftaroline), NHE3 inhibitor (e.g. ceftaroline), nicotinic acid derivative (e.g. ethionamide), NK1 receptor antagonist (e.g. aprepitant), NNRTI (e.g. efavirenz), non-cardioselective beta blocker (e.g. carvedilol), non-sulfonylurea (e.g. metformin), non-steroidal anti-inflammatory drug (e.g. diclofenac), NS5A inhibitor (e.g. daclatasvir), nucleoside reverse transcriptase inhibitor (NRTI) (e.g. tenofovir), nutraceutical product (e.g. omega-3 polyunsaturated fatty acids), oral food supplement (e.g. arginine), other immunostimulants (e.g. glatiramer), other immunosuppressants (e.g. omalizumab), oxazolidinedione anticonvulsant (e.g. trimethadione), oxazolidinone antibiotic (e.g. linezolid), parathyroid hormone and analogues (e.g. teriparatide), PARP inhibitor (e.g. niraparib), PCSK9 inhibitor (e.g. evolocumab), penicillin resistant penicillinase (e.g. oxacillin), peripheral opioid receptor antagonist (e.g. naloxegol), mixed peripheral opioid receptor agonists (egonist/eluxadoline antagonist), peripheral vasodilator (e.g. isoxsuprine), peripherally acting anti-obesity agent (e.g. orlistat), phenothiazine antiemetic (e.g. promethazine), phenothiazine antipsychotic (e.g. prochlorperazine), phenylpiperazine antidepressant (e.g. trazodone), potassium phosphate inhibitor (e.g. trazodone) (e.g. idelalisib), platelet aggregation inhibitor (e.g. aspirin), platelet stimulating agent (e.g. eltrombopag), polyene (e.g. nystatin), potassium-sparing diuretic (e.g. spironolactone), probiotic (e.g. lactobacillus acidophilus), progesterone receptor modulator (e.g. ulipristal), progestin levonorgestrel, prolactin inhibitor (e.g. cabergoline), protease inhibitor (e.g. telaprevir), protease-activated receptor-1 antagonist (e.g. vorapaxar), proteasome inhibitor (e.g. bortezomib), proton pump inhibitor (e.g. omeprazole), psoralen (e.g. methoxsalen), purine nucleoside (e.g. valaciclovir), pyrrolidine anticonvulsant (e.g. levetiracetam), human quinolones (e.g. ciprofloxacin), recombinant human erythropoietin (e.g. epoetin alfa), renin inhibitor (e.g. aliskiren), rifamycin derivative (e.g. rifampicin), salicylate (e.g. aspirin), second generation cephalosporin (e.g. selective cefuroxime receptor), modulator (e.g. ospemifene), selective immunosuppressant (e.g. natalizumab), selective phosphodiesterase-4 inhibitor (e.g. roflumilast), selective serotonin reuptake inhibitor (e.g. escitalopram), serotonin-norepinephrine reuptake inhibitor (e.g. duloxetine), serotonergic neuroenteric (e.g. tegaserod), SGLT-2 inhibitor (e.g. empagliflozin), skeletal muscle relaxant (e.g. onabotulinumtoxinA), smoking quitting agent (e.g. nicotine analog somatostat) (e.g. octreotide), statin (e.g. lovastatin), streptogramin (e.g. dalfopristin/quinupristin), streptomycete derivative (e.g. capreomycin), anticonvulsant succinimide (e.g. ethosuximide), sulfonamide (e.g. sulfamethoxazole), sulphonylurea stimulant (e.g. glimepistole, clomiphene), tetracyclic antidepressant (e.g. mirtazapine), tetracyclines (e.g. minocycline), thiazide diuretic (e.g. hydrochlorothiazide), thiazolidinedione (e.g. pioglitazone), thioxanthene (e.g. thiotixene), third generation cephalosporine (e.g. ceftriaxone), thrombin inhibitor (e.g. dabigatazone), strepolytic (e.g. levothyroxine), TNF alpha inhibitor (e.g. adalimumab), tocolytic agent (e.g. terbutaline), topical acne agent (e.g. tretinoin), topical anesthetic (e.g. lidocaine), topical anti-infectious (e.g. malathion), topical anti-rosacea agent (e.g. ivermectin), topical antibiotic (e.g. silver sulfadiazine), topical antifungal (e.g. econazole), topical antihistamine (e.g. diphenhydramine), topical antineoplastic (e.g. imiquimod), topical anti psoriasis (e.g. tazarotene), topical antivirals (e.g. penciclovir), topical astringent (e.g. hazelnut), topical debridement agent (e.g. collagenase), topical depigmenting agent (e.g. hydroquinone), topical emollient (e.g. emollients), topical keratolytics (e.g. salicylic acid), topical non-steroidal anti-inflammatory (e.g. diclofenac), topical photochemistry (e.g. aminolevulinic acid), topical rubefactive (e.g. menthol), topical steroid (e.g. betamethasone), topical steroid with anti-infectives (e.g. aciclovir/hydrocortisone), transthyretin stabilizer (e.g. tafamidis), anticonvulsant triazine (e.g. lamotrigine), tricyclic antidepressant (e.g. amitriptyline), urea cycle disturbing agent (e.g. sodium phenylbutyrate), urinary anti-infective (e.g. nitrofurantoin), urinary antispasmodic (e.g. amitriptyline) modifier (e.g. potassium citrate), uterotonic agent (e.g. dinoprostone), vaginal anti-infective (e.g. clindamycin), vasodilator (e.g. alprostadil), vasopressin antagonist (e.g. conivaptan), vasopressor (e.g. epinephrine), VEGF/VEGFR inhibitor (e.g. pazopan), viral vaccine, combination of vitamins and minerals, vitamin (e.g. cyanocobalamin), VMAT2 inhibitor (e.g. valbenazine).

The nutritional active ingredient can be of the following types: vitamin (e.g. vitamins A, B, C, D, E, K, folic acid, biotin), mineral (e.g. potassium, chlorine, sodium, calcium, phosphorus, magnesium, iron, zinc, manganese, copper, iodine, chromium, molybdenum, selenium, cobalt, fluoride), amino acid, peptide and protein and their metabolites and derivatives (e.g. essential and branched amino acids, carnosine, enzymes and enzyme complexes, lactoferrin, N-acetylcysteine, proteins animal or vegetable food), fatty acid (e.g. omega-3, omega-6, omega-9 fatty acids), natural product produced using intact sources or substances extracted or derived from plants, animals, algae, fungi, lichens or bacteria (e.g. phytosterol, echinacea, green tea extract, garlic, aloe vera, fish oil, spirulina, chlorella, digestive enzymes derived from mushrooms), sugar and polysaccharides (e.g. mannose, ribose, trehalose, dextrose, glucuronolactone, dextrins), probiotic (e.g. live microorganisms such as Lactobacill us spp, Bifidobacterium spp, Sacc boulardii), prebiotic (e.g. fructans such as fructooligosaccharides and inulins, galactans such as galactooligosaccharides and xylooligosaccharides), antioxidant (e.g. lipoic acid, coenzyme Q10, flavonoids, glutathione, resveratrol, catechins), other substances with a nutritional or physiological effect (e.g. betaine, caffeine, theobromine, theophylline, CDP-choline, choline, creatine, phospholipids, GABA, glucosamine, inositol, melatonin, methylsulfonylmethane, nucleotides, squalene).

In these embodiments, it becomes particularly advantageous to provide that the membrane substrate is made up of one or more biopolymers, the electrospinning of which produces a membrane with a three-dimensional structure such as to receive particles of active ingredient. For example, the three-dimensional structure of the non-woven fabric can contain cavities, also called cages, suitable to house the molecules of active ingredient. One example of a compound for obtaining a non-woven fabric with cavities is a mixture of linear hyaluronic acid with cross-linked hyaluronic acid.

In accordance with some embodiments, regardless of the first electrospun compound, the active ingredient is a non-steroidal anti-inflammatory, to be applied for example on a skin burn. It can also be provided to add one or more analgesics as additional active ingredients, in order to reduce the pain caused by the burn. For this type of application, it is particularly advantageous that the first compound is of the type that is regenerating for the skin, such as for example hyaluronic acid.

The active ingredient can be added to the already electrospun membrane substrate, therefore in its non-woven fabric form, so that molecules or particles of active ingredient are "trapped" inside the three-dimensional structure of the substrate.

Alternatively, the active ingredient can be included in the composition which is then electrospun. In other words, it is possible to provide that the active ingredient is co-electrospun, that is, electrospun together with the material that makes up the substrate. In that case, the resulting patch product comprises the active ingredient integrated into the electrospun fiber.

The use of the patch product according to the invention in the treatment of skin burns is advantageous since it determines a fast absorption of the active ingredient and also of the first compound in the wound. In addition, the product that can be obtained by electrospinning the composition can be applied directly to the burned zone. This improves the effectiveness of the treatment.

As well as treating burns, it should be noted that the patch product according to the invention can be used in any type of application whatsoever, to supply the body with active ingredients in the cosmetic, pharmaceutical or food sector. In particular, the patch product can be advantageously used to apply active ingredients which are currently administered by intramuscular injection. One example are anticoagulants, such as for example heparin. The patch product can also be used for the intake of food supplements.

For this purpose, hyaluronic acid, as a compound to be electrospun, is a good candidate to be combined with different active ingredients, of each of the three types indicated above.

For example, among cosmetic active ingredients we can mention the following: anti-seborrheic (sebacic acid, azelaic acid), antioxidants (ascorbic acid, tocopherol, retinol, retinal), anti-stain (glabridin, ammonium persulfate), emollients (Hamamelis virginiana extract, bisabolol) and humectants (e.g. glycerin, propanediol).

Among the preferred nutritional active ingredients are natural products produced using intact sources or substances extracted or derived from plants, animals, algae, fungi, lichens or bacteria (phytosterol, echinacea, green tea extract, garlic, aloe vera, fish oil, spirulina, chlorella, digestive enzymes derived from fungi), vitamins (e.g. vitamins A, B, C, D, E, K, folic acid, biotin) and antioxidants (e.g. lipoic acid, coenzyme Q10, flavonoids, glutathione, resveratrol, catechins).

The most advantageous pharmaceutical active ingredients are androgens and anabolic steroids (e.g. testosterone), anti-CTLA-4 monoclonal antibodies (e.g. ipilimumab), anti-PD-1 monoclonal antibodies (e.g. nivolumab), antianginal agents (e.g. nitroglycerin), anti-asthma combinations (e.g. diphyllin/guaifenesin), antibiotics (e.g. metronidazole), antibiotics/antineoplastics (e.g. doxorubicin), antineoplastics (e.g. isotretinoin) and antineoplastic combinations (e.g. letrozole/ribociclib).

It should be noted that these active ingredients can be advantageously combined with other compounds to be electrospun such as, for example, xanthan gum, guar gum, chondroitin sulfate, collagen or starch.

Another example of a patch product provides heparin as an electrospun compound and a pharmaceutical active ingredient, for example an allergen extract or a platelet stimulating agent such as eltrombopag.

The patch product according to the invention can also replace already existing patches which provide a membrane substrate that is not absorbable by the skin and that remains attached, sometimes for days, in order to allow the active ingredients to be conveyed into the body. In fact, according to the invention, the membrane substrate is totally absorbed in a short time (typically a few minutes), without having to keep a film attached to the skin.

The present invention also concerns an article that comprises the patch product described above disposed on a support base. Preferably, the support base forms, together with a coating layer placed so as to cover the patch product, a wrapping which then encloses the patch product. To this end, the support base and the coating layer are reciprocally joined so as to conform an internal space, intended to house the patch product.

Preferably, the reciprocal joining occurs by means of heat-sealing, even more preferably it is performed in correspondence with the edges of the support base and the coating layer. The wrapping is able to protect the patch product, that is, the membrane substrate and the one or more active ingredients, isolating it from external agents that could damage it or alter its structure and/or chemical composition.

Favorably, the support base and the coating layer, which can be made of the same material or of two different materials, obviously comprise at least one surface made of a material compatible with the patch product. By compatible with the patch product we mean, in the context of the present description, that the patch product put in contact with this surface is not modified, either chemically or structurally. The surface made of compatible material does not interact with the substrate or with the active ingredient, but acts only as an inert support.

Advantageously, the support base and the coating layer are made of a material of the heat-sealable type, for example polybutylene succinate adipate PBSA or polylactic acid PLA.

A preferred method to produce the article provides to deposit the electrospun membrane substrate directly on the support base. Subsequently, it can be provided to cover the electrospun membrane substrate with the coating layer, and then to join the support base with the coating layer. Advantageously, the reciprocal joining of the support base and the coating layer is performed in such a way as to create an internal space between them, which houses the patch product.

As mentioned above, the reciprocal joining of the layers preferably occurs by means of heat-sealing, even more preferably by means of heat-sealing in correspondence with the edges of the support base and the coating layer.

### EXAMPLES

Different compositions, listed below, were electrospun under the conditions indicated. In the composition examples, the first compound, to be electrospun and which therefore makes up the membrane substrate of the cosmetic patch product, is chosen from the compounds listed in the table below:

| | |
|---|---|
| HA1 | linear hyaluronic acid with average molecular mass equal to 1.2 MDa |
| HA2 | hyaluronic acid oligomer with average molecular mass lower than 10000 Da |
| HA3 | hyaluronic acid with average molecular mass equal to 50000 Da |
| HA4 | cross-linked hyaluronic acid with average molecular mass comprised between 20 and 3000 kDa |

These compounds are supplied by Esperis S.p.A., Milan, Evonik Degussa Italia, Cremona, and IRALAB S.p.A., Usmate Velate (MI). The molecular masses were determined by means of GPC (Gel Permeation Chromatography).

The electrospinning was performed in a NANON.01A apparatus of the Japanese company Mecc CO. Ltd. The experimental conditions are indicated in each of the examples below.

The fibers produced were characterized by means of scanning electron microscopy. In particular, they were coated with gold using an EMITECHK950x Turbo Evaporator sputter coater, EBSciences, East Granby, CT, and observed with a Cambridge Stereoscan 440 SEM, Cambridge, UK scanning electron microscope.

### Examples of electrospinning of compositions comprising hyaluronic acid as a compound to be electrospun and a mixture PEO:alginate as a spinning promoter

The spinning promoter comprises a mixture of an aqueous solution of alginate at 5% by weight with an aqueous solution of PEO at 5% by weight in a proportion of 1:1. The promoter was then mixed with an aqueous solution of linear hyaluronic acid with an average molecular weight of 1.2 MDa (HA1) at 0.5% by weight. The promoter:(HA solution) proportion is equal to 5.6:1. The composition was electrospun with relative humidity (RH) comprised between 24% and 29%, at a temperature of 22°C, with an electric field of 20kV, at a volumetric flow rate of the composition at the head equal to 0.7 mL/h, the distance between the spinning head and the support on which the fiber deposits is equal to 15cm and the needle used being a needle with a diameter 22G. The fiber obtained is regular and has few defects. The same promoter was mixed with an aqueous solution of hyaluronic acid oligomer at 13% by weight, in promoter:(HA solution) proportion equal to 1:3. The electrospinning of this second example of composition, under the same operating conditions as the first example above, has a very regular and defect-free fiber, with an average diameter comprised between 250 and 350 nm. The fiber obtained completely covered the support used.

### Examples of electrospinning of compositions comprising hyaluronic acid as a compound to be electrospun and pullulan as a spinning promoter

The pullulan used is of the food grade type, produced by Hayashibara Co., Ltd. Aqueous solutions of pullulan at 10%, 15% or 20% by weight were prepared, and these aqueous solutions of pullulan (spinning promoter) were mixed with aqueous solutions of hyaluronic acid, for the electrospinning.

The table below lists the examples of compositions that were electrospun, as well as the corresponding operating conditions of the electrospinning.

| | Composition | Electrospinning conditions |
|---|---|---|
| 1 | Pullulan 20%:HA3 29% 1:2 | RH 20-30%, 23kV, 0.1µl/min, 15 cm, needle 22G |
| 2 | Pullulan 20%:HA2 29% 1:2 | RH 46%, T=23°C, 23kV, 1ml/h, 15 cm, needle 22G |
| 3 | Pullulan 10%:HA2 23% 1:3 | RH 40%, T=24°C, 23-25kV, 1ml/h, needle 22G, max distance |
| 4 | Pullulan 10%:HA2 15% 1:2 | RH 49%, T=21°C, 23kV, 0.6ml/h, 18 cm, needle 22G, acid pH (between 1.5 and 3) |
| 5 | (pullulan 15%/alginate 5% 3:1):HA2 23% 1:3 | RH 49%, T=21°C, 23kV, 0.6ml/h, 15 cm, needle 22G |
| 6 | Pullulan 10%:HA2 23% 1:3 | RH 49%, T=21°C, 23kV, 0.15ml/h, 15 cm, needle 22G, acid pH (between 1.5 and 3) |
| 7 | Pullulan 10%:HA2 15% 1:2 | RH 40-50%, T=21°C, 23kV, 0.6ml/h, 15 cm, needle 22G, pH = 5.5 |
| 8 | Pullulan 10%:HA2 23% 1:3 | RH 30%, T=22°C, 23kV, 0.5ml/h, 15 cm, needle 22G, pH = 5.5 |

Example 1 resulted in regular fibers, without defects and with an average diameter from 400 to 700 nm. However, little deposit was observed during the test.

In example 2, the fibers obtained are thick, with an average diameter of 10 µm, due to the high viscosity of the electrospun solution.

In example 3, the fibers obtained have an average diameter comprised between 50 nm and 2 µm. It should be observed that with this example the fibers were deposited both on aluminum and also on a film of PBSA.

Examples 4 and 7 (pullulan:HA ratio equal to 1:2), on the one hand, and 6 and 8 (pullulan:HA ratio equal to 1:3), on the other hand, allowed to verify the effect of the proportions between promoter and hyaluronic acid. In example 4, the solution obtained has optimal properties for a good electrospinning, the fibers obtained have an average diameter ranging from 800 nm to 1 µm. For the composition of example 4, which has an acid pH, the pH was increased up to 5.5 (by adding NaOH 1M) thus obtaining the solution of example 7. With the latter, the electrospinning gave regular and uniform fibers with an average diameter smaller than example 4, between 500 and 700 nm.

By increasing the proportion of hyaluronic acid, in example 6 (with acid pH) fibers with a uniform diameter were obtained, with an average value equal to 1-3 µm, while in example 8 (with pH 5.5) the fibers obtained have a non-uniform diameter ranging from 700 nm to 3 µm.

In example 5, an alginate was added to the pullulan as a stabilizer. With a promoter:HA ratio of 1:3, and under the conditions mentioned in the table, thick fibers were obtained, with an average diameter of the order of several microns.

## Claims

1. Patch product, comprising a membrane substrate absorbable by the skin and at least one active ingredient, **characterized in that** said membrane substrate is formed by at least one electrospun fiber based on a compound selected from xanthan gum, pectin, chitin, chitosan, dextran, carrageenan, guar gum, agar, cellulose derivatives, albumin, starch, gelatin, collagen, elastin, β-glucans, glycosaminoglycans, mucopolysaccharides, water-soluble polysaccharides and their derivatives, and that the active ingredient is selected from cosmetic active ingredients, pharmaceutical active ingredients and nutritional active ingredients, **and in that** said electrospun fiber further comprises pullulan and/or alginate as an electrospinning promoter.

2. Patch product as in claim 1, **characterized in that** the cellulose derivatives are selected from hydroxypropyl methylcellulose, hydroxypropyl cellulose, hydroxyethyl cellulose, sodium carboxymethyl cellulose; the glycosaminoglycans are selected from chondroitin sulfate, dermatan sulfate, heparin, heparan sulfate and hyaluronic acid HA; and/or the water-soluble polysaccharides are selected from galactomannans, xylans, gum arabic, gum ghatti, glucomannan, acemannan, soluble dietary fibers, glycogen, amylose and polysaccharides derived from plants, bacteria and fungi.

3. Patch product as in any claim hereinbefore, **characterized in that** the electrospun compound is selected from starch, elastin, hyaluronic acid, heparin, collagen, pectin, β-glucans, chondroitin sulfate, dermatan sulfate, heparan sulfate and their derivatives.

4. Patch product as in any claim hereinbefore, **characterized in that** the membrane substrate is in the form of a non-woven fabric.

5. Patch product as in any claim hereinbefore, **characterized in that** the electrospun fiber has a diameter smaller than 100µm.

6. Patch product as in any claim hereinbefore, **characterized in that** the active ingredient is integrated in the electrospun fiber.

7. Patch product as in any claim from 1 to 6, **characterized in that** the active ingredient is inserted into the three-dimensional structure of the membrane substrate.

8. Patch product as in any claim hereinbefore, **characterized in that** the active ingredient comprises a non-steroidal anti-inflammatory and/or one or more analgesics.

9. Article comprising a patch product as in any claim hereinbefore disposed on a support base.

10. Article as in claim 9, **characterized in that** it comprises a wrapping inside which the patch product is present, said wrapping comprising the support base and a coating layer that acts as protection for the patch product, wherein said coating layer and said support base are reciprocally joined so as to form an internal space which houses the patch product.

## Patentansprüche

1. Pflasterprodukt, umfassend ein von der Haut absorbierbares Membransubstrat und mindestens einen Wirkstoff, **dadurch gekennzeichnet, dass** das Membransubstrat gebildet ist aus mindestens einer elektrogesponnenen Faser basierend auf einer Verbindung ausgewählt aus Xanthan, Pektin, Chitin, Chitosan, Dextran, Carrageen, Guargummi, Agar, Cellulosederivaten, Albumin, Stärke, Gelatine, Kollagen, Elastin, β-Glucanen, Glykosaminoglykanen, Mucopolysacchariden, wasserlöslichen Polysacchariden und deren Derivaten, und dass der Wirkstoff ausgewählt ist aus kosmetischen Wirkstoffen, pharmazeutischen Wirkstoffen und nährstofflichen Wirkstoffen, und dass die elektrogesponnene Faser ferner Pullulan und/oder Alginat als Elektrospinning-Promotor umfasst.

2. Pflasterprodukt nach Anspruch 1, **dadurch gekennzeichnet, dass** die Cellulosederivate ausgewählt sind aus Hydroxypropylmethylcellulose, Hydroxypropylcellulose, Hydroxyethylcellulose, Natriumcarboxymethylcellulose; die Glykosaminoglykane ausgewählt sind aus Chondroitinsulfat, Dermatansulfat, Heparin, Heparansulfat und Hyaluronsäure HA; und/oder die wasserlöslichen Polysaccharide ausgewählt sind aus Galactomannanen, Xylanen, Gummi arabicum, Gummi ghatti, Glucomannan, Acemannan, löslichen Ballaststoffen, Glykogen, Amylose und Polysacchariden, die aus Pflanzen, Bakterien und Pilzen stammen.

3. Pflasterprodukt nach einem der vorstehenden Ansprüche, **dadurch gekennzeichnet, dass** die elektrogesponnene Verbindung ausgewählt ist aus Stärke, Elastin, Hyaluronsäure, Heparin, Kollagen, Pektin, β-Glucanen, Chondroitinsulfat, Dermatansulfat, Heparansulfat und deren Derivaten.

4. Pflasterprodukt nach einem der vorstehenden Ansprüche, **dadurch gekennzeichnet, dass** das Membransubstrat die Form eines Vliesstoffs aufweist.

5. Pflasterprodukt nach einem der vorstehenden Ansprüche, **dadurch gekennzeichnet, dass** die elektrogesponnene Faser einen Durchmesser von weniger als 100 µm aufweist.

6. Pflasterprodukt nach einem der vorstehenden Ansprüche, **dadurch gekennzeichnet, dass** der Wirkstoff in die elektrogesponnene Faser integriert ist.

7. Pflasterprodukt nach einem der Ansprüche 1 bis 6, **dadurch gekennzeichnet, dass** der Wirkstoff in die dreidimensionale Struktur des Membransubstrats eingebracht ist.

8. Pflasterprodukt nach einem der vorstehenden Ansprüche, **dadurch gekennzeichnet, dass** der Wirkstoff ein nichtsteroidales Antirheumatikum und/oder ein oder mehrere Analgetika umfasst.

9. Artikel, umfassend ein Pflasterprodukt nach einem der vorstehenden Ansprüche, das auf einer Trägerbasis angeordnet ist.

10. Artikel nach Anspruch 9, **dadurch gekennzeichnet, dass** er eine Umhüllung umfasst, in welcher sich das Pflasterprodukt befindet, wobei die Umhüllung die Trägerbasis und eine Beschichtungsschicht, die als Schutz für das Pflasterprodukt dient, umfasst, wobei die Beschichtungsschicht und die Trägerbasis wechselseitig miteinander verbunden sind, um einen Innenraum zu bilden, in dem das Pflasterprodukt aufgenommen ist.

## Revendications

1. Produit de patch, comprenant un substrat membranaire absorbable par la peau et au moins un ingrédient actif, **caractérisé en ce que** ledit substrat membranaire est formé par au moins une fibre électrofilée à base d'un composé choisi parmi la gomme de xanthane, la pectine, la chitine, le chitosane, le dextrane, le carraghénane, la gomme de guar, l'agar, les dérivés de cellulose, l'albumine, l'amidon, la gélatine, le collagène, l'élastine, les β-glucanes, les glycosaminoglycanes, les mucopolysaccharides, les polysaccharides hydrosolubles et leurs dérivés, et **en ce que** l'ingrédient actif est choisi parmi les ingrédients actifs cosmétiques, les ingrédients actifs pharmaceutiques et les ingrédients actifs nutritionnels, **et en ce que** ladite fibre électrofilée comprend en outre du pullulane et/ou de l'alginate en tant que promoteur d'électrofilage.

2. Produit de patch selon la revendication 1, **caractérisé en ce que** les dérivés de cellulose sont choisis parmi l'hydroxypropylméthylcellulose, l'hydroxypropylcellulose, l'hydroxyéthylcellulose, la carboxyméthylcellulose de sodium ; les glycosaminoglycanes sont choisis parmi le sulfate de chondroïtine, le sulfate de dermatane, l'héparine, le sulfate d'héparane et l'acide hyaluronique HA; et/ou les polysaccharides hydrosolubles sont choisis parmi les galactomannanes, les xylanes, la gomme arabique, la gomme ghatti, le glucomannane, l'acémannane, les fibres alimentaires solubles, le glycogène, l'amylose et les polysaccharides dérivés de plantes, de bactéries et de champignons.

3. Produit de patch selon l'une quelconque des revendications précédentes, **caractérisé en ce que** le composé électrofilé est choisi parmi l'amidon, l'élastine, l'acide hyaluronique, l'héparine, le collagène, la pectine, les β-glucanes, le sulfate de chondroïtine, le sulfate de dermatane, le sulfate d'héparane et leurs dérivés.

4. Produit de patch selon l'une quelconque des revendications précédentes, **caractérisé en ce que** le substrat membranaire est sous la forme d'un tissu non tissé.

5. Produit de patch selon l'une quelconque des revendications précédentes, **caractérisé en ce que** la fibre électrofilée a un diamètre inférieur à 100 µm.

6. Produit de patch selon une quelconque des revendications précédentes, **caractérisé en ce que** l'ingrédient actif est intégré dans la fibre électrofilée.

7. Produit de patch selon l'une quelconque des revendications 1 à 6, **caractérisé en ce que** l'ingrédient actif est inséré dans la structure tridimensionnelle du substrat membranaire.

8. Produit de patch selon l'une quelconque des revendications précédentes, **caractérisé en ce que** l'ingrédient actif comprend un anti-inflammatoire non stéroïdien et/ou un ou plusieurs analgésiques.

9. Article comprenant un produit de patch selon l'une quelconque des revendications précédentes disposé sur une base de support.

10. Article selon la revendication 9, **caractérisé en ce qu'**il comprend un emballage à l'intérieur duquel le produit de patch est présent, ledit emballage comprenant la base de support et une couche de revêtement qui sert de protection pour le produit de patch, dans lequel ladite couche de revêtement et ladite base de support sont mutuellement reliées de manière à former un espace interne qui abrite le produit de patch.
